# EUROPÄISCHE PATENTSCHRIFT

(11) **EP 0 832 636 B1**
(45) Veröffentlichungstag und Bekanntmachung des Hinweises auf die Patenterteilung: **19.11.2003**
(21) Anmeldenummer: 97116317.5
(22) Anmeldetag: 19.09.1997
(51) Int. Cl.: A61K 6/083, A61K 6/033

(54) **Polymerisierbarer Dentalwerkstoff und Verwendung von Apatit-Füllstoffen im Dentalwerkstoff**
Polymerisable dental material and use of apatite fillers in the dental material
Matériau dentaire polymérisable et utilisation d'une charge d'apatite dans ce matériau dentaire

(30) Priorität: 30.09.1996 DE 19640454
(43) Veröffentlichungstag der Anmeldung: 01.04.1998
(73) Patentinhaber: DeguDent GmbH, 63457 Hanau (DE)
(72) Erfinder: Rentsch, Harald, Dr., 63457 Hanau (DE)
(74) Vertreter: Stoffregen, Hans-Herbert, Dr. Dipl.-Phys.

(56) Entgegenhaltungen:
- EP-A- 0 710 475
- WO-A-91/12212
- US-A- 4 775 646

## Beschreibung

Die Erfindung beschreibt einen verbesserten polymerisierbaren Dentalwerkstoff mit variabel einstellbarer Transparenz, guter Polierbarkeit, hoher Festigkeit und der Fähigkeit zur Freisetzung und Aufnahme von Ionen in bzw. aus einer biologischen Umgebung. Die Erfindung richtet sich auch auf die. Verwendung von bestimmten Füllstoffen auf Basis von Apatiten im verbesserten polymerisierbaren Dentalwerkstoff.

Insbesondere bezieht sich die Erfindung auf einen Dentalwerkstoff auf Basis von polymerisierbaren, ethylenisch ungesättigten Monomeren als Bindemittel, eines Katalysators für die Kalt-, Heiß- und/oder verschiedene Typen der Photopolymerisation und bezogen auf das Gesamtgewicht des Dentalwerkstoffs 1 - 95 Gewichtsprozent eines anorganischen Füllstoffs, der wenigstens einen bestimmten Apatit-Füllstoff aufweist.

Synthetische Orthophospate, insbesondere Apatite wie Ca₁₀(PO₄)₆(OH)₂ oder Ca₁₀(PO₄)₆F₂ sind aufgrund ihrer chemischen und strukturellen Ähnlichkeit zu biologischen Hartgeweben von besonderem Interesse als Werkstoffe für die Medizin, sowohl allein als auch in Kombination mit anderen Materialien, wie Polymeren, Gläsern usw.

Zum Stand der Technik werden folgende Druckschriften genannt:
(1) US 4,778,834
(2) WO-A 94/23944
(3) Antonucci, J. M., Dent. Mat. 7: 124 - 129, 1991,
(4) EP 0 193 588 B1
(5) EP 0 625 490 A1
(6) DE 39 32 469 C2
(7) DE 23 26 100 B2
(8) DE 25 01 683 A1
(9) US 5,304,577
(10) Derwent-Abstract Nr. 85-021799 [04]
(11) Derwent-Abstract Nr. 85-022140 [04].

(1) beschreibt Dentalmaterialien, welche durch die Kombination von Hydroxylapatit, Ca₁₀(PO₄)₆(OH)₂, in Konzentrationen bis zu 70% mit polymerisierbaren Monomeren hergestellt werden. Die Hydroxylapatit-Partikel werden vor ihrer Einmischung mit einer Silikathülle beschichtet und in der für Dentalwerkstoffe üblichen Art und Weise silanisiert. Nachteilig an diesen Werkstoffen ist, daß der Ionenaustausch (Calcium, Phosphat) mit der Umgebung durch die Silikathülle weitgehend eingeschränkt ist und daß im Werkstoff keine Transparenz erreicht werden kann, wie sie von dentalen Füllungsmaterialen gefordert wird, da der Brechungsindex von Hydroxylapatit, Ca₁₀(PO₄)₆(OH)₂, mit 1,625 zu hoch ist im Vergleich zu den polymeren Komponenten. Dies führt zu opaquen Werkstoffen mit nur geringer Lichtdurchlässigkeit, wodurch die Photopolymerisation dieser Werkstoffe nur in sehr dünnen Schichten möglich ist. Weiterhin ist der Werkstoff nicht zur Freisetzung von Fluorid-Ionen befähigt, welche insbesondere für Dentalwerkstoffe wünschenswert sind, da sie kariespräventiv wirken können.

(2) beschreibt Dentalmaterialien, welche durch die Kombination von gesintertem Fluorapatit, Ca₁₀(PO₄)₆F₂, in Konzentrationen zwischen 10 und 70 Volumenprozent mit polymerisierbaren Monomeren hergestellt werden. Diese Dental-Materialien sind zwar in der Lage, die gewünschten Fluorid-Ionen in ihre Umgebung freizusetzen, sie können jedoch die heutigen Anforderungen an die Transluzenz von Dental-Werkstoffen nicht erfüllen, da der Brechungsindex von Fluorapatit, Ca₁₀(PO₄)₆F₂, mit 1,63 zu hoch ist im Vergleich zu den eingesetzten polymeren Komponenten. Dies führt ebenfalls zu opaquen Werkstoffen mit nur geringer Lichtdurchlässigkeit.

Zur Überwindung dieses Mangels schlägt (3) die Verwendung von Calcium-Metaphosphaten, [Ca(PO₃)₂]ₙ, vor, wodurch eine Verringerung des Brechungsindex auf 1,54-1,59 erzielt werden kann. Mit diesen Füllstoffen lassen sich zwar Dentalwerkstoffe mit der gewünschten Transparenz herstellen, jedoch besitzt dieser Füllstoff im Vergleich zu Fluorapatit eine höhere Löslichkeit und verfügt nicht über die Fähigkeit zur Fluoridabgabe.

Wünschenswert zur Herstellung von dentalen Füllungsmaterialien mit der erforderlichen Transparenz sind daher Füllstoffe, welche die niedrige Löslichkeit und die Fluoridabgabe wie Fluorapatit aufweisen, mit ihrem Brechungsindex jedoch im Bereich der Calcium-Metaphospate liegen.

In der (4) werden Carbonapatit enthaltende Mittel und die Verwendung von Carbonapatit für Implantate beschrieben. Den offenbarten Phosphat/Carbonat-Apatiten mangelt es jedoch an einer zahnähnlichen Transparenz, ebenso wie an einer für den kaudruckbelasteten Bereich bei Zahnfüllungen erforderlichen Festigkeit. Aufgrund der hohen Opazität ist das Material nicht lichthärtend sondern selbsthärtend und folglich nur für den Implantatbereich brauchbar.

Gemäß dem Verfahren der (5) erhältliche carbonathaltige Phosphatapatite verfügen zwar über eine erhöhte Porösität und Transluzenz, allerdings ist der Brechungsindex immer noch unzureichend. In Kombination mit Acrylaten würde ein opakes Material resultieren.

Antimikrobielle Hydroxyapatit-Pulver sind aus der (6) bekannt.

Die (7) beschreibt ebenso wie die (8) bioaktive Verbundmaterialien für prothetische Zwecke, insbesondere zweiphasige Gläser mit einer kristallinen Apatitphase eingebettet in eine Glasphase. Solche Materialien jedoch führen mit den üblichen Acrylat-Harzen unweigerlich zur Trübung. Insbesondere werden Phosphatapatite mit geringen Carbonatanteilen beschrieben, die bekanntermaßen nicht die geforderten optischen Eigenschaften aufweisen. Nachdem auch die offenbarte Glasphase nicht röntgenopaque ist, sind aus den beschriebenen Kombinationen Glasphase/Apatit/Acrylat keine Zahnfüllungsmaterialien zu erwarten.

(9) bezieht sich ausschließlich auf Phosphatapatite. Strontium-Phosphat-Apatit hat ebenso wie Calcium-Phosphat-Apatit einen Brechungsindex von 1,63. Das Problem der Brechungsindex-Erniedrigung läßt sich durch den Austausch von Calcium gegen Strontium nicht lösen. Die Autoren zielen daher auf den Einsatz im nichtsichtbaren Bereich (Zemente für Knochendefekte, Zahnwurzel, Periodont). Insgesamt werden unter den beschriebenen Bedingungen lediglich Dentalwerkstoffe mit hoher Opazität und unzureichender Festigkeit für Füllungen im sichtbaren kaudruckbelasteten Bereich erreicht.

(10) und (11) beschreiben reine Phosphatapatite. In Kombination mit den genannten Dimethacrylaten resultieren opake Pasten. Ihre Opazität ist auch daran erkennbar, daß sie nicht mit Licht des sichtbaren Spectrum ausgehärtet werden können (zu geringe Eindringtiefe des Lichts) sondern das Initiatorsystem (Amin/Peroxid) in ein 2-Pasten-Material verteilt werden muß, welches erst durch Vermischen aktiviert werden kann. Da ausschließlich Apatit als Füller verwendet wird, ist die Festigkeit der resultierenden Materialien zu gering. Die genannten Kationen-Substitutionen als auch die Substitutionen für X führen zu keiner Verringerung des Brechungsindex.

In Anbetracht des hierin angegebenen und diskutierten Standes der Technik war es mithin Aufgabe der Erfindung, einen verbesserten Dentalwerkstoff anzugeben, der alle an einen modernen Dentalwerkstoff gestellten Anforderungen hinsichtlich Transparenz, Polierbarkeit, Druckfestigkeit, Wasseraufnahme, Abriebfestigkeit, Biegefestigkeit, Röntgenopazität usw. erfüllt, der leicht herstellbar und an bestimmte spezielle Erfordernisse leicht anpaßbar ist. Aufgabe der Erfindung ist auch die Angabe von Verwendungen für bestimmte Apatite.

Die Lösung dieser sowie weiterer im einzelnen nicht näher genannter, jedoch aus den einleitenden Erörterungen des Standes der Technik ableitbarer oder sich ohne weiteres erschließender Aufgaben gelingt durch einen Dentalwerkstoff der eingangs erwähnten Gattung, welcher die Merkmale des kennzeichnenden Teils des Anspruches 1 aufweist. Zweckmäßige Weiterbildungen werden in den von Anspruch 1 abhängigen Ansprüchen unter Schutz gestellt. Hinsichtlich der Verwendung bietet Anspruch 18 einen Vorschlag für die Lösung der oben angegebenen Probleme an.

Dadurch, daß die Füllstoff-Komponente des dentalen Werkstoffs
A) einen oder mehrere Mischapatite des Typs

   A₁₀₋ᵣ(XO₄)₆₋ₛZ₂₋ₜ-Bᵣ(Y)ₛQₜ-B'₁₀₋ᵤC_{u'}(Y')₆Z'₂

   worin
   A, B und B' gleich oder verschieden für ein zweiwertiges Kation und/oder eine ladungsäquivalente Kombination ein- und dreiwertiger Kationen stehen, wobei die Kationen Ionenradien im Bereich von 0,069 - 0,174 nm aufweisen,
   X, gleich oder verschieden für drei-, vier-, fünf- und/oder sechswertiges Kation steht, wobei die Kationen Ionenradien im Bereich von 0,026 - 0,056 nm aufweisen,
   Y und Y' unabhängig voneinander gleich oder verschieden (SO₄)²⁻ und/oder (PO₃F)²⁻ ggf. zusammen mit (CO₃)²⁻ bedeuten, wobei der (CO₃)²⁻-Anteil auf höchstens 1/6 beschränkt ist, bezogen auf die Mole an Y und Y',
   Q, Z und Z', unabhängig voneinander gleich oder verschieden für F⁻, OH⁻ und O²⁻ stehen, und
   C für ein einwertiges Kation steht, sowie
   u und u' unabhängig voneinander gleich oder verschieden eine ganze oder gebrochene Zahl im Bereich von 0 bis 6 sind und den Substitutionsgrad von B durch C, im jeweiligen Kationen-Untergitter angeben,
   r eine ganze oder gebrochene Zahl im Bereich von 0 bis 10 ist und den Substitutionsgrad von A durch B im Kationen-Untergitter angibt,
   s eine ganze oder gebrochene Zahl im Bereich von 0 bis 6 ist und den Substitutionsgrad von XO₄ durch Y im Untergitter der tetraedrischen Anionen angilet,
      und
   t eine ganze oder gebrochene Zahl im Bereich von 0 bis 2 ist und den Substitutionsgrad von Z durch Q angibt,
in einer zur Aufnahme von Ionen aus der biologischen Anwendungsumgebung des Dentalwerkstoffs wirksamen Menge aufweist,
gelingt es, einen polymerisierbaren Dentalwerkstoff mit variabel einstellbarer Transparenz, guter Polierbarkeit und hoher Festigkeit zu schaffen, bei dem es unter anderem erreicht wurde:
a) die erforderliche Transparenz einzuhalten;
b) durch eine ausreichend niedrige Löslichkeit der Füllstoffe A) die Bioaktivität durch Fluoridabgabe wie bei einem Fluorapatit zu gewährleisten; und
c) den Brechungsindex des Füllstoffs A) im Bereich der Calcium-Metaphosphate zu halten.

Überraschenderweise konnte gezeigt werden, daß durch die Kombination bestimmter Apatite in Form von Mischkristallen ein Füllstoff hergestellt werden kann, welcher in Kombination mit den für Dentalwerkstoffe gebräuchlichen Monomeren und ggf. anderen Füllstoffen wie Gläsern und Aerosilen die gewünschte Transparenz einstellbar macht, ohne auf die geringe Löslichkeit der Fluorapatite oder die Fähigkeit zur Fluoridabgabe verzichten zu müssen. Der Brechungsindex der Mischapatite ist darüber hinaus innerhalb bestimmter Grenzen variabel einstellbar, wodurch ein genaue Anpassung an die optischen Eigenschaften der verwendeten Monomerkombination möglich ist.

Insbesondere werden von der Erfindung Dentalwerkstoffe mit solchen Mischapatiten umfaßt, für die eine Erniedrigung des Brechungsindex des Mischapatites durch die Mischapatitbildung erreicht wird. Diese Erniedrigung des Brechungsindex ist eine wichtige Voraussetzung dafür, daß die optischen Eigenschaften des gesamten Füllungsmaterials heutigen ästhetischen Ansprüchen genügen können. Nur dadurch ist der optische Fit zum Zahnschmelz in Verbindung mit der Harzmatrix und den anderen, für die Festigkeit des dentalen Füllungswerkstoffes verantwortlichen Füllstoffe möglich.

Die im Stand der Technik bekannten Lösungen liefern diese Leistung nicht, weil sie Mischapatite nur auf der Basis von Phosphatapatit betrachten. Phosphatapatite haben Brechungsindizes ≥ 1,63. Für die erfindungsgemäß beschriebenen dentalen Füllungsmaterialien sind jedoch Werte von < 1,58 unabdingbar. Diese notwendigen Veränderungen der optischen Eigenschaften lassen sich jedoch nur erreichen, wenn deutliche Substitutionen im Untergitter der komplexen Anionen, d. h. der Phosphat-Anionen, vorgenommen werden. Im Rahmen der Erfindung erfolgt dies durch Sulfat-Anionen oder Monofluorphosphat-Anionen, ggf. kombiniert mit bis zu 1/6 (CO₃)²⁻, bezogen auf die gesamten Mole Y und Y'.

Folgerichtig ist die Modifikation der optischen Eigenschaften hin zur Tauglichkeit für die Anwendung im sichtbaren Bereich in keiner der eingangs zitierten Patentschriften das Ziel, und es werden nur Materialien für die Anwendung im nichtsichtbaren Bereich beschrieben (Knochendefekte, Wurzelkanal), die dadurch außerdem nur selbsthärtend als Zwei-Komponentenmaterial herstellbar sind.

Dentalwerkstoff bezeichnet im Rahmen der Erfindung Materialien für die Zahnfüllung, Inlays oder Onlays, Befestigungszemente, Glasionomerzemente, Kompomere, Verblendmaterialien für Kronen und Brücken, Materialien für künstliche Zähne, in Dentinbondings, Unterfüllmaterialien, Wurzelfüllmaterialien oder sonstige Materialien für die prothetische, konservierende und präventive Zahnheilkunde. Insbesondere fallen unter den Begriff Dentalwerkstoff auch Komposits für zahnmedizinische und zahntechnische Einsatzzwecke, Versieglermaterialien, selbsthärtende Komposits, Stumpfaufbaumaterialien, Verblendkunststoffe, hoch- und normalgefüllte Dualzemente sowie normalgefüllte fluoridhaltige Zahnlacke.

Als Bindemittel kommen für den Dentalwerkstoff alle diejenigen Bindemittel auf Basis eines polymerisierbaren, ethylenisch ungesättigten Monomeren in Frage, die dem Fachmann für diesen Einsatzzweck geläufig sind. Zu den mit Erfolg einsetzbaren polymerisierbaren Monomeren gehören vorzugsweise solche mit acrylischen und/oder methacrylischen Gruppen.

Insbesondere handelt es sich hierbei u. a. um Ester der α-Cyanoacrylsäure, (Meth)acrylsäure, Urethan(meth)acrylsäure, Crotonsäure, Zimtsäure, Sorbinsäure, Maleinsäure und Itaconsäure mit ein- oder zweiwertigen Alkoholen; (Meth)acrylamide wie z. B. N-isobutylacrylamid; Vinylester von Carbonsäuren wie z. B. Vinylacetat; Vinylether wie z. B. Butylvinylether; Mono-N-vinyl-Verbindungen wie N-Vinylpyrrolidon; und Styrol sowie seine Derivate. Besonders bevorzugt sind die nachfolgend aufgeführten mono- und polyfunktionellen (Meth)acrylsäureester und Urethan(meth)acrylsäureester.
(a) Monofunktionelle (Meth)acrylate
   Methyl(meth)acrylat, n- oder i-Propyl(meth)acrylat, n-, i- oder tert.-Butyl(meth)acrylat und 2-Hydroxyethyl(meth)acrylat.
(b) Difunktionelle (Meth)acrylate
   Verbindungen der allgemeinen Formel: 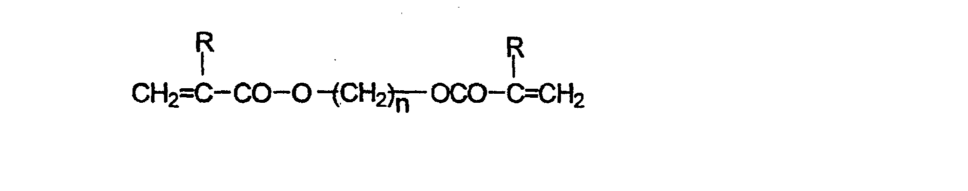 worin R Wasserstoff oder Methyl ist und n eine positive ganze Zahl zwischen 3 und 20, wie z. B. Di(meth)acrylat des Propandiols, Butandiols, Hexandiols, Octandiols, Nonandiols, Decandiols und Eicosandiols, Verbindungen der allgemeinen Formel: 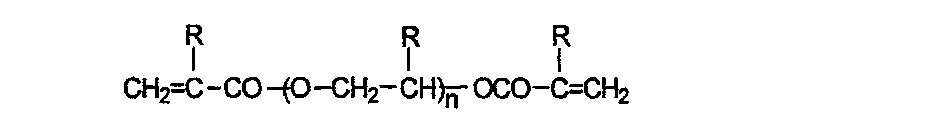 worin R Wasserstoff oder Methyl ist und n eine positive ganze Zahl zwischen 1 und 14, wie z. B. Di(meth)acrylat des Ethylenglycols, Diethylenglycols, Triethylenglycols, Tetraethylenglycols, Dodecaethylenglycols, Tetradecaethylenglycols, Propylenglycols, Dipropylglycols und Tetradecapropylenglycols; und Glycerindi(meth)acrylat, 2,2'-Bis[p-(γ-methacryloxy-β-hydroxypropoxy)-phenylpropan] oder Bis-GMA, Biphenol-A-dimethacrylat, Neopentylglycoldi(meth)acrylat, 2,2'-Di(4-methacryloxypolyethoxyphenyl)propan mit 2 bis 10 Ethoxygruppen pro Molekül und 1,2-Bis(3-methacryloxy-2-hydroxypropoxy)butan.
(c) Tri- oder mehrfachfunktionelle (Meth)acrylate
   Trimethylolpropantri(meth)acrylate und Pentaerythritoltetra(meth)acrylat.
(d) Urethan(meth)acrylat
   Umsetzungsprodukte von 2 Mol hydroxylgruppenhaltigen (Meth)acrylatmonomer mit einem Mol Diisocyanat und Umsetzungsprodukte eines zwei NCO Endgruppen aufweisenden Urethanprepolymers mit einem methacrylischen Monomer, das eine Hydroxylgruppe aufweist, wie sie z. B. durch die allgemeine Formel wiedergegeben werden: 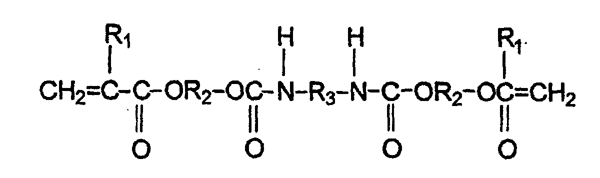
worin R Wasserstoff oder eine Methylgruppe bedeutet, R₂ eine Alkylengruppe und R₃ einen organischen Rest verkörpert.

Die genannten Monomeren werden entweder allein oder in Form einer Mischung von mehreren Monomeren verwendet.

Zu ganz besonders vorteilhaft im erfindungsgemäßen Dentalwerkstoff eingesetzten Monomeren gehören vor allem 2,2-Bis-4(3-methacryloxy-2-hydroxypropoxy)-phenylpropan (Bis-GMA), 3,6-Dioxaoctamethylendimethacrylat (TEDMA) und/oder 7,7,9-Trimethyl-4,13-dioxo-3,14-dioxa-5,12-diazahexadecan-1,16-dioxy-dimethacrylat (UDMA).

Der Dentalwerkstoff kann je nach Art des verwendeten Katalysators heiß, kalt und/oder durch Licht polymerisierbar sein. Als Katalysatoren für die Heißpolymerisation können die bekannten Peroxide wie Dibenzoylperoxid, Dilauroylperoxid, tert.-Butylperoctoat oder tert.-Butylperbenzoat eingesetzt werden, aber auch α,α'-Azo-bis(isobutyroethylester), Benzpinakol und 2,2'-Dimethylbenzpinakol sind geeignet

Als Katalysatoren für die Photopolymerisation können z. B. Benzophenon und seine Derivate sowie Benzoin und seine Derivate verwendet werden. Weitere bevorzugte Photosensibilisatoren sind α-Diketone wie 9,10-Phenanthrenchinon, Diacetyl, Furil, Anisil, 4,4'-Dichlorbenzil und 4,4'-Dialkoxybenzil, Campherchinon wird besonders bevorzugt verwendet. Die Verwendung der Photosensibilisatoren zusammen mit einem Reduktionsmittel wird bevorzugt. Beispiele für Reduktionsmittel sind Amine wie Cyanethylmethylanilin, Dimethylaminoethylmethacrylat, Triethylamin, Triethanolamin, N,N-Dimethylanilin, N-Methyldiphenylamin, N,N-Dimethyl-sym.-xylidin und N,N-3,5-Tetramethylanilin und 4-Dimethylaminobenzoesäureethylester.

Als Katalysatoren für die Kaltpolymerisation werden Radikale liefernde Systeme, z. B. Benzoyl- bzw. Lauroylperoxid zusammen mit Aminen wie N,N-Dimethyl-sym.xylidin oder N,N-Dimethyl-p-toluidin verwendet. Es können auch dual härtende Systeme zur Katalyse verwendet werden, z. B. Photoinitiatoren mit Aminen und Peroxiden. Als Photokatalysatoren kommen auch Mischungen aus UV-lichthärtenden und im Bereich des sichtbaren Lichts härtenden Katalysatoren in Betracht.

Die Menge dieser Katalysatoren im Dentalwerkstoff liegt üblicherweise zwischen 0,01 bis 5 Gew.-%.

Vorzugsweise dient der erfindungsgemäße Dentalwerkstoff als Zahnfüllungsmaterial. Zahnfüllungsmaterialien werden auch als Zweikomponentenmaterialien hergestellt, die nach dem Anmischen kalt aushärten. Die Zusammensetzung ist ähnlich wie bei den lichthärtenden Materialien, nur wird anstatt der Photokatalysatoren in die eine Paste z. B. Benzoylperoxid und in die andere Paste z. B. N,N-Dimethylp-toluidin eingearbeitet. Durch Vermischen etwa gleicher Teile der beiden Pasten erhält man ein Zahnfüllungsmaterial, welches in wenigen Minuten aushärtet.

Wenn man bei den letztgenannten Materialien das Amin wegläßt und als Katalysator z. B. nur Benzoylperoxid verwendet, erhält man einen heißhärtenden Dentalwerkstoff, der für die Herstellung eines Inlays bzw. von künstlichen Zähnen verwendet werden kann. Für die Herstellung eines Inlays wird im Mund des Patienten von der Kavität ein Abdruck genommen und ein Gipsmodell hergestellt. In die Kavität des Gipsmodells wird die Paste eingebracht und das Ganze wird in einem Drucktopf unter Hitze polymerisiert.

Das Inlay wird entnommen, bearbeitet und dann im Munde des Patienten in die Kavität einzementiert.

Der Füllstoff A) ist ein essentieller und charakteristischer Bestandteil des erfindungsgemäßen Dentalwerkstoffs.

Beim Füllstoff A) handelt es sich in jedem Fall um wenigstens einen sogenannten Mischapatit. Mischapatit bezeichnet dabei eine chemische Mischung von zwei oder drei Apatiten, d. h. zwei oder drei Apatite haben ein gemeinsames Kristallgitter.

Der u. a. erfindungsgemäß erstrebte Effekt, nämlich der Austausch von Ionen mit der biologischen Umgebung des Dentalwerkstoffs bei gleichzeitig hoher Transparenz und Härte des Dentalwerkstoffs läßt sich durch verschiedene Typen von Mischapatiten erreichen.

So kann die Erfindung durch gefüllte Dentalwerkstoffe realisiert werden, die Mischapatite des Typs A) aufweisen. Zum Erfolg führen ebenso Füllstoffmischungen A), welche 2 oder mehr Mischapatite des Typs A) aufweisen.

Die Mischapatite des Typs A) entsprechen der allgemeinen Formel

A₁₀₋ᵣ(XO₄)₆₋ₛZ₂₋ₜ-B₂(Y)ₛQₜ-B'₁₀₋ᵤC_{u'}(Y')₆Z'₂

worin
A, B und B' gleich oder verschieden für ein zweiwertiges Kation und/oder eine ladungsäquivalente Kombination ein- und dreiwertiger Kationen stehen, wobei die Kationen Ionenradien im Bereich von 0,069 - 0,174 nm aufweisen,
X, gleich oder verschieden für drei-, vier-, fünf- und/oder sechswertiges Kation steht, wobei die Kationen Ionenradien im Bereich von 0,026 - 0,056 nm aufweisen,
Y und Y' unabhängig voneinander gleich oder verschieden (SO₄)²⁻ und/oder (PO₃F)²⁻ ggf. Zusammen mit (CO₃)²⁻ bedenten, wobei der (CO₃)²⁻-Anteil auf höchstens 1/6 beschränkt ist, bezogen auf die Mole an Y and Y'
Q, Z und Z' unabhängig voneinander gleich oder verschieden für F⁻, OH⁻ und O²⁻ stehen, und
C für ein einwertiges Kation steht
   sowie
u und u' unabhängig voneinander gleich oder verschieden eine ganze oder gebrochene Zahl im Bereich von 0 bis 6 sind und den Substitutionsgrad von B durch C im jeweiligen Kationen-Untergitter angeben,
r eine ganze oder gebrochene Zahl im Bereich von 0 bis 10 ist und den Substitutionsgrad von A durch B im Kationen-Untergitter angibt,
s eine ganze oder gebrochene Zahl im Bereich von 0 bis 6 ist und den Substitutionsgrad von XO₄ durch Y im Untergitter der tetraedrischen Anionen angibt,
   und
t eine ganze oder gebrochene Zahl im Bereich von 0 bis 2 ist und den Substitutionsgrad von Z durch Q bzw angilet.

Bei dem Apatit des Typs A) gilt, daß die gewählte Schreibweise verdeutlichen soll, aus welchen einzelnen Apatiten das Kristallgitter des jeweiligen Mischapatits zusammengesetzt gedacht werden kann.

In zweckmäßiger Ausgestaltung weist der Dentalwerkstoff Mischapatite des Typs A) auf , worin A, B und/oder B' gleich oder verschieden Mg²⁺, Ca²⁺, Sr²⁺, Ba²⁺, Cu²⁺, Ag²⁺, Zn²⁺, Na⁺ und/oder K⁺, sind.

Insbesondere hat es sich im Rahmen der Erfindung auch gezeigt, daß bei Einbau bestimmter Kationen in die erfindungsgemäßen Mischapatite eine autimikrobielle Wirkung erzielbar ist. Besonders zweckmäßig in diesem Zusammenhang ist der Einbau von Cu²⁺, Ag²⁺, Zn²⁺, und/oder Sn²⁺.

Dabei ist die erfindungsgemäße Lösung des Einbaus in den Mischapatit deutlich günstiger, als die vorbekannte Möglichkeit mikrobiell wirkende Mittel an Apatite anzulagern, wie dies z.B. aus der DE 39 32 469 bekannt ist.

Ferner ist es für den erfindungsgemäßen Dentalwerkstoff bevorzugt, wenn er Füllstoffe A) aufweist, worin X, gleich oder verschieden B³⁺, Al³⁺, Si⁴⁺, P⁵⁺ und/oder S⁶⁺ sind.

In noch einer anderen zweckmäßigen Ausführungsform kennzeichnet sich der Dentalwerkstoff der Erfindung durch Füllstoffe A) worin C, Na⁺ bedeutet.

Der Füllstoff A), ist erfindungsgemäß im Dentalwerkstoff in einer Menge enthalten, die ausreicht, einen Ionenaustausch mit der biologischen Umgebung zu erlauben. Vorteilhaft ist es, wenn der Gewichtsanteil an Füllstoff A) 1 bis 70 Gew.-%, bezogen auf den Dentalwerkstoff, beträgt. Besonders zweckmäßig ist eine Abwandlung, worin der Gewichtsanteil an Füllstoff A) 2 bis 35 Gew.-%, bezogen auf das Gesamtgewicht des Dentalwerkstoffs, beträgt. Ganz besonders günstig erweist es sich, wenn der Gewichtsanteil an Füllstoff A) 2 bis 7 Gew.-%, bezogen auf den Dentalwerkstoff, beträgt.

Im Rahmen dieser Mengen kann über eine größere Vielfalt an Zusammensetzungen und Mengen der Brechungsindex des Dentalwerkstoffs gezielt eingestellt werden. In bevorzugter Ausführungsform kennzeichnet sich dieser dadurch, daß der Brechungsindex der Füllstoffkomponente A) im Bereich von 1,50 bis 1, 66 liegt.

Die Partikelgröße der im Dentalwerkstoff gemäß der Erfindung einzusetzenden Füllstoffe A) ist über weite Bereiche unkritisch. Vorzugsweise kennzeichnet sich die Erfindung dadurch, daß die Partikelgröße der Füllstoffkomponente A) im Bereich von 0,01 bis 10 µm ist.

Die Herstellung der Apatite erfolgt auf dem Fachmann geläufige Weise, z. B. durch Festkörperreaktion nach literaturbekannten Verfahren (s. z.B. A.G. Cockbain,: Miner. Mag. 1968,**36**, 654).

Durch die Variation der Edukte lassen sich auf diese Weise Mischapatite mit einem Brechungsindex zwischen 1, 63 und 1,52 einstellen.

Insgesamt kann gesagt werden, daß die erfindungsgemäßen Mischapatite auf SO₄²⁻ - und PO₃F²⁻ -Anionen beruhen, die einen untergeordneten Anteil, nämlich bis zu 1/6 der Mole, an CO₃²⁻ -Anionen aufweisen können.

Wegen seiner planaren Struktur ist (CO₃)²⁻ nur sehr begrenzt zur Substitution tetraedrischer Phosphat-Gruppen geeignet. Als Folge sind die Einflußmöglichkeiten zur Senkung des Brechungsindex von Phosphat-Apatiten nur gering. Im Gegensatz dazu ist mit dem tetraedrischen Sulphat- bzw. Monofluorphosphat-Ion ein vollständiger Ersatz der Phosphat-Gruppen möglich mit entsprechend großem Einfluß auf die Brechungsindizes der modifizierten Mischapatite.

Daneben können die Füllstoffe auch in silanisierter Form eingesetzt werden. Hierdurch wird eine bessere Einbindung in die Polymermatrix erreicht.

Neben der essentiellen und charakteristischen Füllstoffkomponente A) kann der Dentalwerkstoff weitere Füllstoffe B) und C) als optionale aber dennoch sehr vorteilige und nicht minder bevorzugte Komponenten aufweisen.

Somit kennzeichnet sich die Erfindung in einer noch weiteren bevorzugten Ausführungsform dadurch, daß der Dentalwerkstoff in Mischung mit der Komponente A) als Füllstoffkomponente: B)
B1) asphärische, splitterförmige Pulver aus Quarz,
   Glaskeramik und/oder Glas mit einem Brechungsindex von 1,46 bis 1,58 und einer durchschnittlichen Teilchengröße von 0,5 bis 5,0 µm
   und/oder
B2) Mikrokugeln
   und/oder
B3) anorganische Fasern
   und/oder
B4) organische Fasern
   aufweist.

Bevorzugt ist dabei das Gewichtsverhältnis von A) zu B) im Bereich von 10 : 1 bis 1 : 30.

Bei dem anorganischen Füllstoff B1) des Füllstoffgemisches handelt es sich um Quarz-, Glaskeramik- oder Glaspulver. Bevorzugt werden Gläser verwendet. Die durchschnittliche Primärteilchengröße des anorganischen Füllstoffes B1) soll zwischen 0,5 und 5,0 pm , insbesondere zwischen 1,0 und 2,0 µm und besonders bevorzugt zwischen 1,0 und 1,5 µm liegen, während der Brechungsindex Werte zwischen 1,50 und 1,58, insbesondere zwischen 1,52 und 1,56 aufweisen soll. Es können auch Füllstoffgemische eingesetzt werden. Bevorzugt werden erfindungsgemäß Ba-Silikatgläser mit einer mittleren Korngröße im Bereich von 1,1 bis 1,3 µm, sowie Sr-Silikatgläser mit einer mittleren Korngröße im Bereich von 1,1 bis 1,3 µm, sowie Li/Al-Silikatgläser mit einer mittleren Korngröße von 1,0 bis 1,6 µm verwendet. Solche Pulver lassen sich z. B. durch Feinmahlung mit einer herkömmlichen Ultrafeinstmühle erhalten.

Als splitterförmige Füllstoffe vom Typ B) kommen auch B5) Polysiloxan-Füllstoffe in Frage, die Aluminium-haltig sind und aus Einheiten der Formel 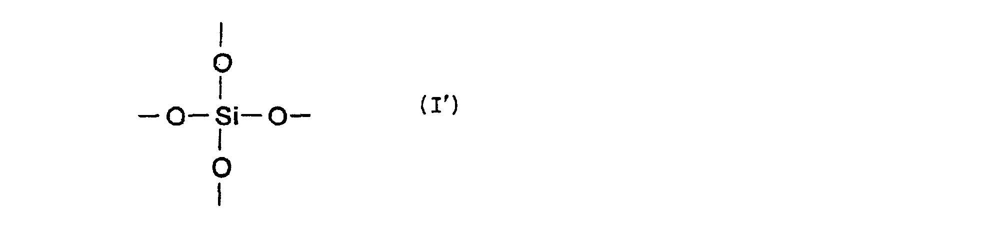 und Einheiten der Formel 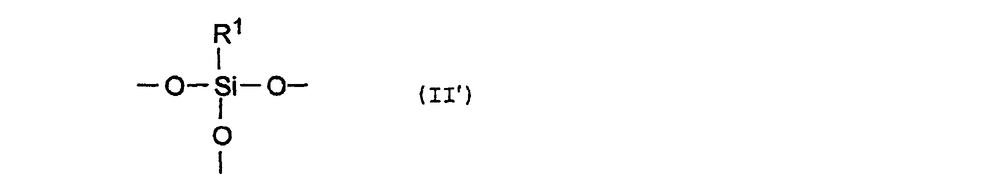 wobei R¹ für eine mit einem Acrylat- oder Methacrylatrest verbundene lineare oder verzweigte Alkylgruppe mit 1 bis 6 C-Atomen oder für einen einfach olefinisch ungesättigten linearen oder verzweigten Kohlenwasserstoffrest mit 2 bis 8 C-Atomen oder für einen zyklischen, einfach olefinisch ungesättigten Kohlenwasserstoffrest mit 5 - 8 C-Atomen oder für eine lineare oder verzweigte Alkylgruppe mit 1 bis 8 C-Atomen, eine Cycloalkylengruppe mit 5 bis 8 C-Atomen, eine Phenylgruppe oder eine Alkylarylgruppe steht, und/oder Einheiten der Formel 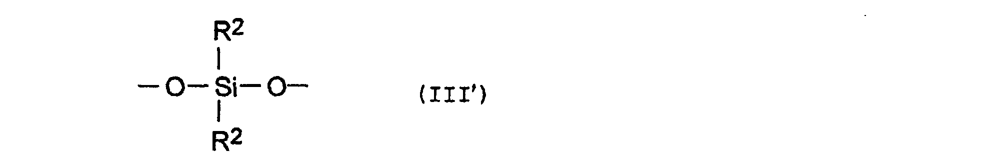 in denen R² eine Methyl-, Ethyl-, Propyl- oder Phenylgruppe repräsentiert, besteht und - bei jeder der Zusammensetzungen - Einheiten der Formel 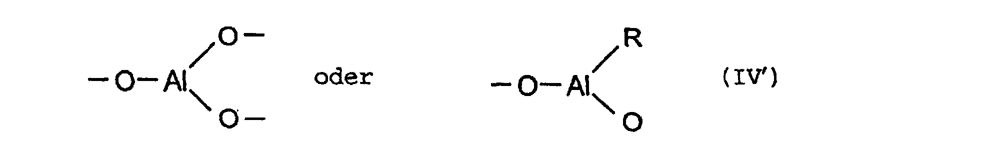 in denen R³ eine lineare oder verzweigte Alkylgruppe mit 1 bis 5 C-Atomen oder eine Phenylgruppe ist, vorliegen und die freien Valenzen der an die Silicium- und Aluminiumatome gebundenen Sauerstoffatome bei den Einheiten (I'), (II') und/oder (III') sowie (IV') wie bei Heterosiloxangerüsten durch ein Siliciumatom einer gleichen oder einer unterschiedlichen Einheit oder durch ein Aluminiumatom abgesättigt werden, wobei das Verhältnis der Siliciumatome aus den Einheiten der Formel (I') zu der Summe der Siliciumatome der Einheiten (II') und (III') 3 : 1 bis 100 : 1 und das Verhältnis der Summe der Siliciumatome aus den Einheiten (I'), (II') und (III') zu den Aluminiumatomen aus den Einheiten (IV') 2 1 bis 200 : 1 beträgt.

Dabei kann das Aluminium im Polysiloxan-Füllstoff ggf. auch durch ein anderes Metall ersetzt sein.

Weitere Füllstoffe vom Typ B) sind u. a. B2) Mikrokugeln, wie sie beispielsweise in DE-A 32 47 800 beschrieben sind, B3) anorganische Fasern, B4) organische Fasern. Diese können allein, in Mischung von zwei oder mehreren und/oder zusammen mit Quarz-, Glaskeramik- oder Glaspulvern B1) oder Polysiloxan-Füllstoffen vom Typ B5) eingesetzt werden.

Zu den Füllstoffen B2) gehört u.a. ein amorphes, kugelförmiges Material auf der Basis von Siliciumdioxid, das daneben noch ein Oxid mindestens eines Metalles der Gruppen I, II, III und IV des Periodensystems enthält. Bevorzugt wird Strontium- und/oder Zirkonoxid verwendet. Die durchschnittliche Primärteilchengröße liegt im Bereich von 0,1 bis 1,0 µm, insbesondere bei 0,15 bis 0,5 µm. Der Brechungsindex des anorganischen Füllstoffs B2) liegt zwischen 1,50 und 1,58, insbesondere zwischen 1,52 und 1,56. Ein besonders bevorzugter Wert ist 1,53 ± 0,01. Es sind auch Füllstoffmischungen möglich, vorausgesetzt, sie erfüllen die Parameter hinsichtlich Teilchengröße und Brechungsindex. Der Füllstoff des Typs B2) kann auch gesintert als Mischung von Agglomeraten mit einer durchschnittlichen Teilchengröße von 1 bis 30 µm vorliegen.

Zu den Füllstoffen B3) gehören u.a. Glasfasern, Glasfasernetze, die gegebenenfalls gesintert sind, Aluminiumoxidfasern und ähnliche.

Zu den Füllstoffen B4) zählen neben anderen Polyethylenfasern und Carbonfasern.

In einer anderen vorteiligen Variante ist der Dentalwerkstoff der Erfindung dadurch gekennzeichnet, daß er neben Füllstoff A) als Füllstoff C) im fertig auspolymerisierten Dentalwerkstoff kugelförmige auf SiO₂ basierende Partikel aufweist aus
C1) einem sphärischen, polydispersen und/oder monodispersen SiO₂-Kern, der an individuellen Punkten seiner Oberfläche mit Metalloxidpartikeln beschichtet ist, wobei die beschichteten Partikel einen Brechungsindex von 1,45 bis 1,62 und eine durchschnittliche Primärteilchengröße von 0,005 bis 2 µm aufweisen und die Beschichtungspartikel aus Metalloxid eine durchschnittliche Größe von weniger als 60 nm besitzen, und/oder
C2) SiO₂ mit einem Brechungsindex von zwischen ca. 1,38 und < 1,50 und einer durchschnittlichen Primärteilchengröße von ca. 0,04 µm bis 1,5 µm,
   und/oder
C3) einem SiO₂-Kern, der mit einem Oxid mindestens eines Elements der Gruppen I, II, III und IV des Periodensystems beschichtet ist, wobei die beschichteten Partikel einen Brechungsindex von 1,45 bis 1,62 und eine durchschnittliche Primärteilchengröße von 0,04 bis 1,5 µm aufweisen und die Beschichtung zwischen ca. 15 und 40 nm dick ist,
   und/oder
C4) unter C1), C2) oder C3) beschriebenen Partikeln, die zusätzlich mit einer Schicht aus einem polymerisierbaren organischen Bindemittel überzogen sind, welches auf mono- oder mehrfachfunktionellen (Meth)acrylaten und/oder Reaktionsprodukten aus Isocyanaten und OH-gruppenhaltigen Methacrylaten beruht, wobei die Primärteilchengröße der mit polymerisierbarer Bindemittelschicht versehenen Partikel zwischen ca. 0,04 und 1,5 µm liegt, während die Schichtdicke der Überzugsschicht im Bereich von 5 nm bis 50 nm und der Brechungsindex der beschichteten Partikel im Bereich von 1,40 - 1,52 liegt.

Dabei liegt das Gewichtsverhältnis von A) zu C) vorzugsweise im Bereich von 10:1 bis 1 : 30.

Ein im erfindungsgemäßen Dentalwerkstoff einzusetzender Füllstoff C1) basiert auf reinen SiO₂-Partikeln. Aus diesen lassen sich dann die an speziellen Oberflächenstellen beschichteten SiO₂-Partikel erhalten.

Die SiO₂-Ausgangspartikel sind polydispers, monodispers und können auch als Mischung von polydispersen und monodispersen Sphären vorliegen.

Bevorzugt sind die SiO₂-Ausgangspartikel monodispers, unporös und essentiell kugelförmig. Grundsätzlich sind alle Oxid-Partikel geeignet, die sich durch hydrolytische Polykondensation aus Alkoholatverbindungen entsprechender Elemente erhalten lassen und dabei in Form monodisperser kompakter sphärischer Partikel anfallen. Die grundliegenden Reaktionsbedingungen zur Herstellung von SiO₂-Partikeln durch hydrolytische Polykondensation sind beispielsweise aus den Publikationen W. Stöber et al. in J. Colloid and Interface Science 26, 62 (1968) und 30, 568 (1969) sowie dem US-Patent 3,634,588 zu entnehmen.

Für die Herstellung von hochmonodispersen, unporösen, kugelförmigen SiO₂-Partikeln, die eine Standardabweichung von nicht mehr als 5 % aufweisen, wird auf EP 0 216 278 hingewiesen, die ein entsprechend abgestelltes Herstellungsverfahren auf Basis von hydrolytischer Polykondensation offenbart. Kern dieses Verfahrens, das für die Herstellung der SiO₂-Partikel gemäß vorliegender Erfindung ganz besonders bevorzugt wird, ist eine zweistufige Verfahrensweise. Hierbei wird zunächst durch hydrolytische Polykondensation von Tetraalkoxysilanen in wäßrig-alkalisch-ammoniakalischem Medium ein Sol bzw. eine Suspension von Primärteilchen gebildet, die man daran anschließend durch dosierte Zugabe von weiterem Tetraalkoxysilan auf die gewünschte Endgröße bringt.

Ein sinngemäßes Verfahren zur Herstellung verschiedener Metalloxide in Form sphärischer Partikel mit enger Teilchengrößenverteilung ist ebenfalls aus EP 0 275 688 zu entnehmen.

An individuellen Stellen oder Punkten mit Partikeln aus Metalloxid beschichtete sphärische SiO₂-Partikel, bei denen die "beschichteten" Partikel einen Brechungsindex im Bereich von 1,45 bis 1,62 und eine durchschnittliche Primärteilchengröße von 0,005 bis 2 µm aufweisen und die Beschichtungspartikel eine durchschnittliche Partikelgröße von weniger als 60 nm besitzen, sind beispielsweise dadurch erhältlich, daß man an sich bekannte, hoch monodisperse, nicht poröse, sphärische SiO₂-Partikel mit einer Standardabweichung von nicht mehr als 5 % bei einer Temperatur im Bereich von 50 - 90 °C in einer Konzentration von 1 bis 30 Gewichtsprozent in entionisiertem Wasser dispergiert, eine wäßrige 5 bis 40 gewichtsprozentige Metallsalzlösung, bevorzugt Titansalzlösung, bei einem pH-Wert im Bereich von 1,3 bis 2,5 und einer Zugaberate im Bereich von 0,0005 bis 0,5 mg Metalloxid, bevorzugt TiO₂, pro Minute und pro m² Oberfläche der SiO₂-Partikel hinzufügt, während der pH-Wert durch Zugabe einer Base im angegebenen Bereich gehalten wird, die solcherart beschichteten SiO₂-Partikel abtrennt, mit Wasser und dann mit Alkohol, bevorzugt Ethanol wäscht, zunächst an der Luft und dann im Vakuum bei Temperaturen im Bereich von etwa 70 bis 125 °C trocknet und gegebenenfalls calciniert.

Alternativ hierzu sind im erfindungsgemäßen Dentalwerkstoff als Füllstoffkomponente C1) verwendbare an individuellen Punkten oder Stellen ihrer Oberfläche eine Beschichtung mit Metalloxidpartikeln, wobei die Beschichtungspartikel aus Metalloxid im beschichteten Partikel eine durchschnittliche Partikelgröße von weniger als 60 nm besitzen, aufweisende sphärische SiO₂-Partikel mit einem Brechungsindex im Bereich von 1,45 bis 1,62 und einer durchschnittlichen Primärteilchengröße im Bereich von 0,005 bis 2 µm auch dadurch erhältlich, daß man eine wäßrige Lösung eines Metallhalogenids, bevorzugt Titantetrachlorid, unter Rühren auf etwa 60 °C erwärmt, die erhaltene Metalloxidsuspension, im bevorzugten Fall TiO₂-Suspension, tropfenweise zu einer Suspension sphärischer SiO₂-Partikel (erhältlich wie oben beschrieben) zugibt, den pH-Wert mit conc, bevorzugt 32 %iger, NaOH-Lösung unter Rühren auf etwa 2,0 einstellt, ein Silankopplungsmittel zur Suspension gibt, nach etwa 15 Minuten den pH-Wert mit conc. NaOH auf etwa 8,0 anhebt, die Suspension nochmals 10 Minuten lang rührt, die abfiltrierten, gewaschenen und getrockneten SiO₂-Partikel mit einer Mischvorrichtung pulverisiert und das erhaltene Pulver bei 700 °C 5 Minuten lang calciniert.

Die Primärteilchengröße der an individuellen Stellen beschichteten sphärischen SiO₂-Partikel, welche als Füllstoff C1) dienen, liegt für die erfindungsgemäßen Dentalwerkstoffe üblicherweise im Bereich zwischen 0,005 und 2 µm. Bevorzugt ist eine Größe der Primärteilchen, d. h. der den beschichteten SiO₂-Sphären zugrundeliegenden SiO₂-Kugeln, von etwa 0,05 bis 0,5 µm. Ganz besonders bevorzugt ist im Sinne der Erfindung eine Primärteilchengröße von 0,1 bis 0,5 µm. Für gewisse Dentalanwendungen zweckmäßig ist jedoch auch der Bereich von 0,01 bis 0,2 µm, vorzugsweise von 0,05 bis 0,15 µm.

Unter anderem nach den geschilderten Verfahren sind beschichtete SiO₂-Partikel erhältlich, bei denen die Beschichtungspartikel Oxide mindestens eines Elements der Gruppen I, II, III und/oder IV des Periodensystems sind. Hiervon sind Oxide der Gruppe IV des Periodensystems bevorzugt. Zweckmäßig sind vor allem Oxide des Titans oder Zirkoniums. Möglich sind aber auch Oxide aus der Eisengruppe ebenso wie Oxide aus der Gruppe der Lanthaniden vorteilhaft sein können. Besonders günstig gestalten sich die Füllstoffe C1) aber auch dann, wenn die Oxide TiO₂, Fe₂O₃ und/oder ZrO₂ sind.

Insgesamt gesehen hat sich eine Beschichtung mit TiO₂ äußerst gut bewährt.

Insbesondere läßt die Wahl des zur Beschichtung eingesetzten Metalloxids unter anderem die zielgerichtete Beeinflussung und Einstellung des Brechungsindex des Füllstoffs C1) und damit auch die Anpassung an den Brechungsindex des gesamten Dentalwerkstoffs zu.

Die Größe der Metalloxidpartikel, vorzugsweise der TiO₂, Fe₂O₃ und ZrO₂-Partikel, im Füllstoff C1) liegt im Bereich von weniger als 60 nm. Vorzugsweise beträgt die durchschnittliche Partikelgröße der an einzelnen Stellen der Oberfläche der SiO₂-Primärteilchen nach Art einer Beschichtung vorhandenen Metalloxidpartikel zwischen 0,0005 und 50 nm.

Der Anteil der Metalloxide in den Füllstoffen C1) liegt im Rahmen der Erfindung zwischen etwa 20 und 75 Gewichtsprozent, vorzugsweise zwischen 40 und 50 Gewichtsprozent, jeweils bezogen auf das Gesamtgewicht des Füllstoffs C1). In besonders vorteiliger Ausgestaltung der Erfindung kennzeichnet sich der Dentalwerkstoff gemäß der Erfindung dadurch, daß bei den Partikeln C1) das Metalloxid Gewichtsverhältnis von Beschichtungspartikel aus Metalloxid zu Kern aus SiO₂ im Bereich von 1 : 4 bis 3 : 1 ist, besonders bevorzugt im Bereich von 1 : 2 bis 2 : 1.

In zweckmäßiger Ausführungsform weisen die Partikel C1) mit strukturierter Oberfläche eine spezifische Oberfläche > 150 m²/g (BET) sowie eine durchschnittliche Porengröße von 5 nm auf. Sie können, was nachfolgend beschrieben wird, in einer für Dentalwerkstoffe üblichen Form silanisiert werden.

Weiters ist es möglich, den Füllstoff C1) aus SiO₂-Sphären und Metalloxidpartikelbeschichtung nachzubeschichten. Hierzu werden organische oder anorganische Substanzen nach an sich bekannten Methoden eingesetzt. Durch die Nachbeschichtung wird es noch sicherer möglich, eine eventuelle Agglomeration der monodispersen Partikel während des Trocknungsprozesses zu vermeiden.

In besonderer Ausführungsform ist der Dentalwerkstoff dadurch gekennzeichnet, daß die Partikel C1) mit Silanen der allgemeinen Formel RSi(OX)₃, worin R eine Alkylgruppe mit 1 bis 18 C-Atomen und X eine Alkylgruppe mit 1 oder 2 C-Atomen ist, und/oder Metalloxiden nachbeschichtet sind. Wenn zur Nachbeschichtung ein Silan verwendet wird, ist es zweckmäßig, wenn dies in einer Menge von etwa 0,02 bis 2 Gewichtsprozent des Silans, berechnet als SiO₂, angewendet wird, bezogen auf das Gewicht der Partikel C1). Ähnlich können auch die Füllstoffe A silanisiert werden. Werden Metalloxide zur Nachbeschichtung eingesetzt, dann geschieht dies in einer Menge von 1 bis 100 Gewichtsprozent, bevorzugt 10 Gewichtsprozent, bezogen auf den Metalloxidgehalt der nicht nachbeschichteten Partikel C1).

Zu besonders vorteiligen Nachbeschichtungsagenzien gehören unter anderem CH₃Si(OMe)₃, TiO₂, Fe₂O₃ und/oder ZrO₂.

Zweckmäßig ist auch eine Ausführungsform, in der die Partikel C1) zusätzlich mit einer Schicht aus einem polymerisierbaren organischen Bindemittel überzogen sind, welches auf mono- oder mehrfachfunktionellen (Meth)acrylaten und/oder Reaktionsprodukten aus Isocyanaten und OH-gruppenhaltigen Methacrylaten beruht, wobei die Primärteilchengröße der mit polymerisierbarer Bindemittelschicht versehenen Partikel zwischen ca. 0,04 und 1,5 µm liegt, während die Schichtdicke der Überzugsschicht im Bereich von 5 nm bis 50 nm und der Brechungsindex der beschichteten Partikel im Bereich von 1,40 - 1,52 liegt.

Als Füllstoffe C) kommen neben den Füllstoffen C1) auch die Varianten C2) bis C4) allein, in Zumischung zu C1) oder auch beliebige Mischungen von C1), C2), C3) und/oder C4) in Frage.

Daher entspricht es einer bevorzugten Ausgestaltung des erfindungsgemäßen Dentalwerkstoffs, wenn dieser neben den Partikeln C1) als Füllstoffkomponente C) Partikel aufweist aus
C2) SiO₂ mit einem Brechungsindex von zwischen ca. 1,38 und < 1,50 und einer durchschnittlichen Primärteilchengröße von ca. 0,04 µm bis 1,5 µm,
C3) einem SiO₂-Kern, der mit einem Oxid mindestens eines Elements der Gruppen I, II, III und IV des Periodensystems beschichtet ist, wobei die beschichteten Partikel einen Brechungsindex von 1,45 bis 1,62 und eine durchschnittliche Primärteilchengröße von 0,04 bis 1,5 µm aufweisen und die Beschichtung zwischen ca. 15 und 40 nm dick ist,
   und/oder
C4) unter C1), C2) oder C3) beschriebenen Partikeln, die zusätzlich mit einer Schicht aus einem polymerisierbaren organischen Bindemittel überzogen sind, welches auf mono- oder mehrfachfunktionellen (Meth)acrylaten und/oder Reaktionsprodukten aus Isocyanaten und OH-gruppenhaltigen Methacrylaten beruht, wobei die Primärteilchengröße der mit polymerisierbarer Bindemittelschicht versehenen Partikel zwischen ca. 0,04 und 1,5 µm liegt, während die Schichtdicke der Überzugsschicht im Bereich von 5 nm bis 50 nm und der Brechungsindex der beschichteten Partikel im Bereich von 1,40 - 1,52 liegt.

Den Komponenten C2) bis C4) ist dabei insbesondere gemeinsam, daß alle Sorten auf SiO₂ basieren oder ausschließlich daraus bestehen und ferner alle Komponenten C2) bis C4) nicht als Agglomerat sondern als separate Partikel im auspolymerisierten Dentalwerkstoff vorliegen, ebenso wie die Komponente C1).

Erfindungsgemäß können z. B. als Komponente C2) SiO₂-Partikel mit einem Brechungsindex von zwischen 1,38 und < 1,50 mit einer durchschnittlichen Primärteilchengröße von ca. 0,04 µm bis ca. 1,5 µm als Komponente C) eingesetzt werden, bevorzugt zusammen mit C1).

In bevorzugter erfindungsgemäßer Ausführungsform weisen die als Komponente C2) dem Füllstoff zumischbaren reinen SiO₂-Partikel einen durchschnittlichen Teilchendurchmesser von ca. 0,04 µm bis ca. 0,25 µm auf. Diese besondere Größe eignet sich vor allem in Dentalwerkstoffen, die speziell bei Füllungswerkstoffen, Verblendwerkstoffen oder künstlichen Zähnen Anwendung finden.

Insbesondere auch für diese Anwendungszwecke ist es besonders zweckmäßig, daß die durchschnittliche Teilchengröße der reinen SiO₂-Partikel insgesamt nicht größer als 0,1 µm ist.

Dort wo die Transparenz des Werkstoffes von untergeordneter Bedeutung ist (beispielsweise bei Stumpfaufbaumaterialien, Zementen für die Befestigung, Versieglern und dgl.), sind auch Partikelgrößen von 0,25 µm bis 1,5 µm bevorzugt.

Besonders zweckmäßig liegen die Teilchen dann in einer Größe von 0,25 bis 1,0 µm vor.

Die erfindungsgemäß als Komponente C2) zu verwendenden reinen SiO₂-Partikel sind bevorzugt monodispers, unporös und essentiell kugelförmig. Grundsätzlich sind alle Oxid-Partikel geeignet, die sich durch hydrolytische Polykondensation aus Alkoholatverbindungen entsprechender Elemente erhalten lassen und dabei in Form monodisperser kompakter sphärischer Partikel anfallen. Die grundlegenden Reaktionsbedingungen zur Herstellung von SiO₂-Partikeln durch hydrolytische Polykondensation sind am oben angegebenen Ort beschrieben.

Für die Herstellung von hochmonodispersen, unporösen, kugelförmigen SiO₂-Partikeln, die eine Standardabweichung von nicht mehr als 5 % aufweisen, wird ebenfalls auf die bereits oben zitierten Quellen verwiesen.

Bei den Füllstoffen vom Typ C3) handelt es sich im Rahmen der Erfindung um schichtförmige Partikel, die einen SiO₂-Kern aufweisen, der mit einem Oxid mindestens eines Elementes der Gruppen I, II, III und IV des Periodensystems beschichtet ist.

Hierbei kann das als Kern verwendete SiO₂-Partikel grundsätzlich mit dem unter C2) beschriebenen reinen SiO₂-Partikel identisch sein, i. d. R. weist es jedoch eine um die Dicke der Beschichtung verminderte Teilchengröße als Ausgangsprodukt auf. Es ist wesentlich hervorzuheben, daß die Partikel vom Typ C3) erfindungsgemäß nicht als Mischoxid-Partikel vorliegen, sondern vielmehr der SiO₂-Kern von einem entsprechenden anderen Oxid mindestens eines Elements der Gruppen I bis IV des Periodensystems umgeben ist. Es hat sich überraschenderweise herausgestellt, daß im Gegensatz zu den Mischoxiden die beschichteten Partikel i. d. R. separat und insbesondere nicht aggregiert im auspolymerisierten Dentalwerkstoff vorliegen. Die beschichteten Partikel vom Typ C3) lassen sich grundsätzlich in Analogie zu den unter C2) hierin beschriebenen zweistufigen Verfahren herstellen. Dabei wird bei der hydrolytischen Polykondensation von Tetraalkoxysilan in wässrig-alkalisch-ammoniakalischem Medium ein Sol bzw. eine Suspension von Primärteilchen gebildet, die man daran anschließend durch dosierte Zugabe eines anderen Tetraalkoxysilans auf die gewünschte Endgröße bringt. So wird also ein Primärpartikel aus einem Oxid gebildet, auf das dann im Aufwachsschritt ein anderes Oxid oder ein Oxidgemisch abgeschieden wird. Hierdurch läßt sich vorteilhaft der resultierende Brechungsindex variieren. Würde die Menge des im Aufwachsschritt gebildeten Oxides in dem fertigen Partikel überwiegen, so wäre es für den resultierenden Brechungsindex im wesentlichen verantwortlich. Zu den besonders bevorzugten auf den SiO₂-Kern abgeschiedenen oxidischen Verbindungen der Gruppen I bis IV des Periodensystemes gehören u. a. TiO₂, ZrO₂, Al₂O₃ und/oder V₂O₅. Dabei ist TiO₂ ganz besonders bevorzugt, wobei bei dessen Verwendung darauf zu achten ist, daß das TiO₂ vollständig gebunden ist, da sonst ggf. Gelbfärbungen des Dentalwerkstoffes auftreten können. Neben den bevorzugten Verbindungen aus den Gruppen I bis IV des Periodensystems sind auch andere Verbindungen möglich. So kann man auch mit Vorteil Nb₂O₅ oder Mischsysteme mit den vorgenannten Oxiden aus den Gruppen I bis IV des Periodensystems mit Erfolg auf SiO₂-Primärpartikeln abscheiden.

Die unter C1), C2) oder C3) beschriebenen Partikel können erfindungsgemäß zusätzlich mit einer Schicht aus einem polymerisierbaren organischen Bindemittel überzogen sein (Füllstoff C4)), welches auf mono- oder mehrfachfunktionellen Methacrylaten und/oder Reaktionsprodukten aus Isocyanaten und OH-Gruppen-haltigen Methacrylaten basiert. Damit ist eine für den jeweiligen Anwendungszweck ggf. vorteilhafte organische Modifizierung der Partikel an der Oberfläche möglich. Diese kann in völliger Übereinstimmung mit Methoden vorgenommen werden, wie sie für die Herstellung von als chromatographische Sorbentien gebräuchlichen Kieselgelen bekannt sind. Gängige Modifizierungsmittel sind dabei Organotrialkoxysilane, wie z. B. Methyltriethoxysilan, Ethyltriethoxysilan, Octyltriethyoxysilan, Octadecyltriethoxysilan, Mono-. oder Polyfluoroalkylethoxysilan oder auch Silane mit funktionalisierten Organogruppen, die eine spätere weitere Modifizierung durch kovalente Bindungsknüpfung in bekannter Weise ermöglichen. Im letzteren Fall sind solche Organotrialkoxysilane im Hinblick auf den erfindungsgemäßen Einsatz der Partikel als Füllstoffe in polymeren oder polymerisierbaren Systemen bevorzugt, die solche funktionelle Gruppen aufweisen, mit denen sich eine kovalente Einbindung in das Polymermaterial erreichen läßt. Beispiele hierfür sind Trimethoxyvinylsilan, Triethoxyvinylsilan und 3-Glycidoxypropyltrimethoxysilan, sowie Silane mit Hydroxyl-, Carboxyl-, Epoxy- und Carbonsäureestergruppen tragenden anorganischen Resten. Die Einbindung der solcher Art modifizierten Partikel gemäß A4) in den Dentalwerkstoff geschieht dabei durch Einarbeitung der Partikel in den Dentalwerkstoff und anschließende Polymerisation bei der eigentlichen Aushärtung des Dentalwerkstoffes.

Alternativ hierzu ist es auch möglich, die oberflächenmodifizierten Partikel gemäß C1), C2) oder C3) vor der eigentlichen Einarbeitung in den Dentalwerkstoff zu polymerisieren. Dies kann beispielsweise in Übereinstimmung mit einem Verfahren geschehen, welches im Journal of Colloid and Interface Science 160, 298 - 303 (1993) beschrieben ist. In jedem Falle kann durch geeignete Abstimmung des zur Oberflächen-Modifizierung verwendeten Monomeren auf das Monomer oder die Monomermischung, welche die Polymermatrix des Dentalwerkstoffes ausbildet, eine Feinabstufung und Regulierung des Brechungsindex des gesamten Dentalwerkstoffes erzielt werden.

Gegebenenfalls können noch weitere Füllstoffe (D) zur Erzielung einer erhöhten Röntgenopazität eingesetzt werden, wobei deren mittlere Primärteilchengröße 5,0 µm nicht übersteigen sollte. Solche Füllstoffe sind z. B. in der DE-OS 35 02 594 beschrieben.

Gegebenenfalls können zur Einstellung der Viskosität geringe Mengen an mikrofeiner, pyrogener oder naßgefällter Kieselsäure (Füllstoff (E)) in den Dentalwerkstoff eingearbeitet werden, höchstens jedoch 50 Gew.-%, bezogen auf den Dentalwerkstoff. Bevorzugt sind 5 - 25, besonders bevorzugt 5 - 12 Gew.-%.

Im Rahmen der Erfindung ist es weiterhin zweckmäßig, das Verhältnis von Füllstoff B) zu Füllstoff C) so im fertigen Dentalwerkstoff einzustellen, daß eine Aggregatbildung der Füllstoffe (C) vermieden wird. Dies ist vom Fachmann durch entsprechende Versuche empirisch zu ermitteln.

In besonders bevorzugter Ausführungsform wird das Verhältnis der Füllstoffkomponenten (C) zu den Füllstoffen (B) im Bereich von 1 : 85 bis 4 : 1 (jeweils bezogen auf Gew.-%) so eingestellt, daß die Festigkeit des resultierenden Dentalwerkstoffes bezogen auf seine Druckfestigkeit im Bereich von > 320 bis ca. 480 MPa beträgt. Hierbei hat es sich als besonders günstig herausgestellt, wenn das Verhältnis von Füllstoffen C) zu Füllstoffen B) ≥ 1 : 10 beträgt (jeweils bezogen auf Gew.-%).

Gegenstand der Erfindung ist auch Verwendung von einem oder mehreren Mischapatiten des Typs

A₁₀₋ᵣ(XO₄)₆₋ₛ Z₂₋ₜ-Bᵣ(Y)ₛQₜ-B'₁₀₋ᵤC_{u'} (Y')₆Z'₂

worin
A, B und B' gleich oder verschieden für ein zweiwertiges Kation und/oder eine ladungsäquivalente Kombination ein- und dreiwertiger Kationen stehen, wobei die Kationen Ionenradien im Bereich von 0,069 - 0,174 nm aufweisen,
X gleich oder verschieden für drei-, vier-, fünf- und/oder sechswertiges Kation steht, wobei die Kationen Ionenradien im Bereich von 0,026 - 0,056 nm aufweisen,
Y und Y' unabhängig voneinander gleich oder verschieden (SO₄)²⁻ und/oder (PO₃F)²⁻ ggf. zusammen mit (CO₃)²⁻ bedeuten, wobei der (CO₃)²⁻ -Anteil auf höchstens 1/6 beschränkt ist, bezogen auf die Mole an Y und Y'
Q, Z und Z' unabhängig voneinander gleich oder verschieden für F⁻, OH⁻ und O²⁻ stehen, und
C für ein einwertiges Kation steht,
sowie
u und u' unabhängig voneinander gleich oder verschieden eine ganze oder gebrochene Zahl im Bereich von 0 bis 6 sind und den Substitutionsgrad von B durch C im jewiligen Kationen-Untergitter angeben,
r eine ganze oder gebrochene Zahl im Bereich von 0 bis 10 ist und den Substitutionsgrad von A durch B im Kationen-Untergitter angibt,
s eine ganze oder gebrochene Zahl im Bereich von 0 bis 6 ist und den Substitutionsgrad von XO₄ durch Y im Untergitter der tetraedrischen Anionen angibt,
   und
t eine ganze oder gebrochene Zahl im Bereich von 0 bis 2 ist und den Substitutionsgrad von Z durch Q angilet,
   in einem Dentalwerkstoff auf Basis von polymerisierbaren, ethylenisch ungesättigten Monomeren als Bindemittel, eines Katalysators für die Kalt-, Heiß- und/oder Photopolymerisation und bezogen auf den Dentalwerkstoff 1 - 95 Gew.-% eines anorganischen Füllstoffs in einer zur Aufnahme von Ionen aus der biologischen Anwendungsumgebung wirksamen Menge.

Die in der Anmeldung beschriebenen Füllungswerkstoffe können aufgrund ihrer hervorragenden optischen Eigenschaften im sichtbaren Bereich der Mundhöhle und damit im direkten Kontakt zum Speichel eingesetzt werden. Der Apatitanteil der Füllung kann damit an den Speichel und an den umgebenden Zahnschmelz Ionen abgeben (u. a. Fluorid, Phosphat, Calcium) und auch aufnehmen. Letzteres kann auch aus anderen externen Quellen geschehen, wie Zahnpasta.

Nachfolgend wird die Erfindung anhand von Ausführungsbeispielen und Vergleichsbeispielen weitergehend erläutert.

### Beispiele

Zur Herstellung der nachfolgend beschriebenen Pasten wurde ein handelsüblicher Kneter der Fa. Grieser verwendet. Seine Knetwerkzeuge wurden so modifiziert, daß eine besonders intensive Durchmischung und homogene Verteilung der Ausgangsstoffe möglich war. Die Homogenisierung der Monomermischungen erfolgte üblicherweise über einen Dreiwalzenstuhl.

Bei lichthärtenden Systemen betrug die Aushärtungszeit 40 sec mit einer handelsüblichen Lampe (Degulux der Firma Degussa).

### Methodenbeschreibung:

### Brechungsindex:

Die Bestimmung des Brechungsindex der feinkörnigen pulverförmigen Proben erfolgte mittels Einbettungsmethode unter einem Lichtmikroskop des Typs JENAPOL INTERPHAKO (ZEISS). Die Pulverprobe wird in eine Flüssigkeit mit bekanntem Brechungsindex eingebettet. An der Phasengrenze entsteht eine helle Linie (BECKEsche Line), welche beim Heben des Tubus in das höher brechende Medium wandert. Der Brechungsindex der flüssigen Phase wird variiert bis

Übereinstimmung mit der Pulverprobe erreicht ist.

### Transparenz:

Die Bestimmung der Transparenz erfolgt an Probekörpern der Dicke d=3,0±0,1 mm und einem Durchmesser von 20±0,1 mm. Zur Herstellung der Proben wird die in die Stahlformen gleicher Dimension eingefüllte Kompositpaste mit 400kp für 30 sec belastet und anschließend für 2 min mit einer Dentallampe der Lichtintensität von mindestens 200 rel. Einheiten ausgehärtet. Während des Aushärtevorgangs ist die Kompositoberfläche durch eine transparente Folie vom Luftsauerstoff abgeschirmt. Die Messung der Transparenz erfolgt mit einem UV/VIS Spektrophotometer PU8800 (Philips) in Transmissionsmodus.

### Beispiel 1

In 22,52g eine Monomermischung, bestehend aus 45 Teilen Bis-GMA, 20 Teilen UDMA und Teilen 35 TRGDMA, werden 68,14g silanisiertes Barium-Silikatglas; 5g Aerosile und 4g Ca₁₀(PO₄)₆F₂, n_{D}=1,63, mit 0,034 Gew.% Campherchinon eingearbeitet. Die entstehende Paste wird mit Licht (Degulux®) ausgehärtet. Es entsteht eine weiße, opaque Paste, welche nach der Aushärtung eine Transparenz von TR = 18,6% (770nm) aufweist.

### Beispiel 2

In 22,52g eine Monomermischung, bestehend aus 45 Teilen Bis-GMA, 20 Teilen UDMA und Teilen 35 TRGDMA, werden 68,14g silanisiertes Barium-Silikatglas, 5g Aerosile und 4g Ca₈Na₂[(PO₄)₄(SO₄)₂]F₂, n_{D} = 1,57, mit 0, 034 Gew.% Campherchinon eingearbeitet. Die entstehende Paste wird mit Licht (Degulux®) ausgehärtet. Es entsteht eine weiße, transparente Paste, welche nach der Aushärtung eine Transparenz von TR = 24,30% (770nm) aufweist. Die Erhöhung der Transparenz um 6% reicht aus, um die Paste durch das Einkneten von Pigmenten in einer gewünschten Zahnfarbe einfärben zu könne, ohne dadurch die zahnschmelz-ähnliche Transparenz einzubüßen, wie in Beispiel 1.

Dieses Verhalten gilt für alle erfindungsgemäßen Dentalwerkstoffe.

## Patentansprüche

1. Dentalwerkstoff auf Basis von polymerisierbaren, ethylenisch ungesättigten Monomeren als Bindemittel, eines Katalysators für die Kalt-, Heiß- und/oder Photopolymerisation und bezogen auf den Dentalwerkstoff 1 - 95 Gew.-% eines anorganischen Füllstoffs,
**dadurch gekennzeichnet, dass**
der Füllstoff A) einen oder mehrere Mischapatite des Typs
A₁₀₋ᵣ(XO₄)₆₋ₛZ₂₋ₜ - Bᵣ(Y)ₛQₜ - B'₁₀₋ᵤC_{u'}(Y')₆Z'₂
worin A, B und B' gleich oder verschieden für ein zweiwertiges Kation und/oder eine ladungsäquivalente Kombination ein- und dreiwertiger Kationen stehen, wobei die Kationen Ionenradien im Bereich von 0,069 - 0,174 nm aufweisen,
X gleich oder verschieden für drei-, vier-, fünf- und/oder sechswertige Kationen steht, wobei die Kationen Ionenradien im Bereich von 0,026 - 0,056 nm aufweisen,
Y und Y' unabhängig voneinander gleich oder verschieden (SO₄)²⁻ und/oder (PO₃F)²⁻ ggf. zusammen mit (CO₃)²⁻ bedeuten, wobei der (CO₃)²⁻-Anteil auf höchstens 1/6 beschränkt ist, bezogen auf die Mole an Y und Y',
Q, Z und Z' unabhängig voneinander gleich oder verschieden für F⁻, OH⁻ und O²⁻ stehen und
C für ein einwertiges Kation steht
sowie
u und u' unabhängig voneinander gleich oder verschieden eine ganze oder gebrochene Zahl im Bereich von 0 bis 6 sind und den Substitutionsgrad von B durch C im jeweiligen Kationen-Untergitter angeben,
r eine ganze oder gebrochene Zahl im Bereich von 0 bis 10 ist und den Substitutionsgrad von A durch B im Kationen-Untergitter angibt,
s eine ganze oder gebrochene Zahl im Bereich von 0 - 6 ist und den Substitutionsgrad von XO₄ durch Y im Untergitter der tetraedrischen Anionen angibt, und
t eine ganze oder gebrochene Zahl im Bereich von 0 - 2 ist und den Substitutionsgrad von Z durch Q angibt,
in einer zur Aufnahme von Ionen aus der biologischen Anwendungsumgebung des Dentalwerkstoffes wirksamen Menge aufweist.

2. Dentalwerkstoff nach Anspruch 1,
**dadurch gekennzeichnet,**
**daß** der Brechungsindex der Füllstoffkomponente A) im Bereich von 1,50 bis 1,66 liegt.

3. Dentalwerkstoff nach Anspruch 1 oder 2 ,
**dadurch gekennzeichnet,**
**daß** die Partikelgröße der Füllstoffkomponente A) im Bereich von 0,01 bis 10 µm ist.

4. Dentalwerkstoff nach einem oder mehreren der vorhergehenden Ansprüche,
**dadurch gekennzeichnet, dass**
A, B und/oder B' gleich oder verschieden Mg²⁺, Ca²⁺, Sr²⁺, Ba²⁺, Cu²⁺, Ag²⁺, Zn²⁺, Na⁺ und/oder K⁺ sind.

5. Dentalwerkstoff nach einem oder mehreren der vorhergehenden Ansprüche
**dadurch gekennzeichnet, dass**
X gleich und/oder verschieden P⁵⁺, Si⁴⁺, B³⁺, Al³⁺ und/oder S⁶⁺ ist.

6. Dentalwerkstoff nach einem oder mehreren der vorhergehenden Ansprüche
**dadurch gekennzeichnet, dass**
C Na⁺ bedeutet.

7. Dentalwerkstoff nach einem oder mehreren der vorhergehenden Ansprüche,
**dadurch gekennzeichnet,**
**daß** der Gewichtsanteil an Füllstoff A) 1 bis 70 Gew.-%, bezogen auf den Dentalwerkstoff, beträgt.

8. Dentalwerkstoff nach einem oder mehreren der vorhergehenden Ansprüche,
**dadurch gekennzeichnet,**
**daß** der Gewichtsanteil an Füllstoff A) 2 bis 35 Gew.-%, bezogen auf den Dentalwerkstoff, beträgt.

9. Dentalwerkstoff nach einem oder mehreren der vorhergehenden Ansprüche,
**dadurch gekennzeichnet,**
**daß** der Gewichtsanteil an Füllstoff A) 2 bis 7 Gew.-%, bezogen auf den Dentalwerkstoff, beträgt.

10. Dentalwerkstoff nach einem oder mehreren der vorhergehenden Ansprüche,
**dadurch gekennzeichnet,**
**daß** er in Mischung mit der Komponente A)
B1) asphärischen, splitterförmigen Pulvern aus Quarz, Glaskeramik und/oder Glas mit einem Brechungsindex von 1,46 bis 1,58 und einer durchschnittlichen Teilchengröße von 0,5 bis 5,0 µm
und/oder
B2) Mikrokugeln
und/oder
B3) anorganische Fasern
und/oder
B4) organische Fasern
aufweist.

11. Dentalwerkstoff nach Anspruch 10,
**dadurch gekennzeichnet,**
**daß** das Gewichtsverhältnis von A) zu B) im Bereich von 10 : 1 bis 1 : 30 ist.

12. Dentalwerkstoff nach einem oder mehreren der vorhergehenden Ansprüche,
**dadurch gekennzeichnet,**
**daß** er als Füllstoff C) im fertig auspolymerisierten Dentalwerkstoff kugelförmige auf SiO₂ basierende Partikel aufweist aus
C1) einem sphärischen, polydispersen und/oder monodispersen SiO₂-Kern, der an individuellen Punkten seiner Oberfläche mit Metalloxidpartikeln beschichtet ist, wobei die beschichteten Partikel einen Brechungsindex von 1,45 bis 1,62 und eine durchschnittliche Primärteilchengröße von 0,005 bis 2 µm aufweisen und die Beschichtungspartikel aus Metalloxid eine durchschnittliche Größe von weniger als 60 nm besitzen,
und/oder
C2) SiO₂ mit einem Brechungsindex von zwischen ca. 1,38 und < 1,50 und einer durchschnittlichen Primärteilchengröße von ca. 0, 04 µm bis 1, 5 µm, und/oder
C3) einem SiO₂-Kern, der mit einem Oxid mindestens eines Elements der Gruppen I, II, III und IV des Periodensystems beschichtet ist, wobei die beschichteten Partikel einen Brechungsindex von 1,45 bis 1,62 und eine durchschnittliche Primärteilchengröße von 0,04 bis 1,5 µm aufweisen und die Beschichtung zwischen ca. 15 und 40 nm dick ist,
und/oder
C4) unter C1), C2) oder C3) beschriebenen Partikeln, die zusätzlich mit einer Schicht aus einem polymerisierbaren organischen Bindemittel überzogen sind, welches auf mono- oder mehrfachfunktionellen (Meth)acrylaten und/oder Reaktionsprodukten aus Isocyanaten und OH-gruppenhaltigen Methacrylaten beruht, wobei die Primärteilchengröße der mit polymerisierbarer Bindemittelschicht versehenen Partikel zwischen ca. 0,04 und 1,5 µm liegt, während die Schichtdicke der Überzugsschicht im Bereich von 5 nm bis 50 nm und der Brechungsindex der beschichteten Partikel im Bereich von 1,40 - 1,52 liegt.

13. Dentalwerkstoff nach Anspruch 12,
**dadurch gekennzeichnet,**
**daß** das Gewichtsverhältnis von A) zu C) im Bereich von 10:1 bis 1 : 30 ist.

14. Dentalwerkstoff nach einem oder mehreren der vorhergehenden Ansprüche 10 bis 13,
**dadurch gekennzeichnet,**
**daß** das Verhältnis von Füllstoff (B) zu Füllstoff (C) so eingestellt ist, daß eine Aggregatbildung der Füllstoffe (C) vermieden wird.

15. Dentalwerkstoff nach einem der vorhergehenden Ansprüche 10 bis 14,
**dadurch gekennzeichnet,**
**daß** das Verhältnis von (C) zu (B) im Bereich von 1 : 85 bis 4 : 1 so eingestellt wird, daß die Festigkeit des Dentalwerkstoffes bezogen auf seine Druckfestigkeit von > 320 bis 480 MPa beträgt.

16. Dentalwerkstoff nach einem oder mehreren der vorhergehenden Ansprüche 10 bis 15,
**dadurch gekennzeichnet,**
**daß** das Verhältnis von (C) zu (B) ≥ 1 : 10 beträgt.

17. Dentalwerkstoff nach einem oder mehreren der vorhergehenden Ansprüche 10 bis 16,
**dadurch gekennzeichnet,**
**daß** die Partikel C) im fertig auspolymerisierten Dentalwerkstoff separat als diskrete Teilchen vorliegen.

18. Verwendung von einem oder mehreren Mischapatiten des Typs
A₁₀₋ᵣ(XO₄)₆₋ₛZ₂₋ₜ - Bᵣ(Y)ₛQₜ - B₁₀₋ᵤCu'(Y')₆Z'₂
worin A, B und B' gleich oder verschieden für ein zweiwertiges Kation
und/oder eine ladungsäquivalente Kombination ein- und dreiwertiger Kationen stehen, wobei die Kationen Ionenradien im Bereich von 0,069 - 0,174 nm aufweisen,
X gleich oder verschieden für drei-, vier-, fünf- und/oder sechswertige Kationen steht, wobei die Kationen Ionenradien im Bereich von 0,026 - 0,056 nm aufweisen,
Y und Y' unabhängig voneinander gleich oder verschieden (SO₄)²⁻ und/oder (PO₃F)²⁻ ggf. zusammen mit (CO₃)²⁻ bedeuten, wobei der (CO₃)²⁻ -Anteil auf höchstens 1/6 beschränkt ist, bezogen auf die Mole an Y und Y',
Q, Z und Z' unabhängig voneinander gleich oder verschieden für F⁻, OH⁻ und O²⁻ stehen und
C für ein einwertiges Kation steht
sowie
u und u' unabhängig voneinander gleich oder verschieden eine ganze oder gebrochene Zahl im Bereich von 0 bis 6 sind und den Substitutionsgrad von B durch C im jeweiligen Kationen-Untergitter angeben,
r eine ganze oder gebrochene Zahl im Bereich von 0 bis 10 ist und den Substitutionsgrad von A durch B im Kationen-Untergitter angibt,
s eine ganze oder gebrochene Zahl im Bereich von 0 - 6 ist und den Substitutionsgrad von XO₄ durch Y im Untergitter der tetraedrischen Anionen angibt, und
t eine ganze oder gebrochene Zahl im Bereich von 0 - 2 ist und den Substitutionsgrad von Z durch Q angibt,
in einem Dentalwerkstoff auf Basis von polymerisierbaren, ethylenisch ungesättigten Monomeren als Bindemittel, eines Katalysators für die Kalt-, Heißund/oder Photopolymerisation und bezogen auf den Dentalwerkstoff 1-95 Gew.-% eines anorganischen Füllstoffs in einer zur Aufnahme von Ionen aus der biologischen Anwendungsumgebung wirksamen Menge.

## Claims

1. Dental material based on polymerisable, ethylenically unsaturated monomers as binding agent, a catalyst for the cold, hot and/or photopolymerization and, relative to the dental material, 1 - 95 wt.% of an inorganic filler,
**characterized in**
**that** the filler A) comprises one or more mixing apatites of the type
A₁₀₋ᵣ(XO₄)₆₋ₛZ₂₋ₜ - Bᵣ(Y)ₛQₜ - B'₁₀₋ᵤC_{u'}(Y')₆Z'₂
in which A, B and B' are the same or different and stand for a bivalent cation and/or a charge-equivalent combination of monovalent or trivalent cations, whereby the cations have ionic radii within the range of 0.069 - 0.174 nm,
X is the same or different and stands for cations of valency three, four, five or six, whereby the cations have ionic radii within the range of 0.026 - 0.056 nm,
Y and Y' independently of one another are the same or different and signify (SO₄)²⁻ and/or (PO₃F)²⁻ optionally together with (CO₃)²⁻, whereby the proportion of (CO₃)²⁻ is restricted to at most 1/6, relative to the mol of Y and Y',
Q, Z and Z' independently of one another are the same or different and stand for F⁻, OH⁻ and O²⁻ and
C stands for a monovalent cation
and also
u and u' independently of one another are the same or different and are an integer or fraction within the range from 0 to 6 and specify the degree of substitution of B by C in the respective cation sublattice,
r is an integer or fraction within the range from 0 to 10 and specifies the degree of substitution of A by B in the cation sublattice,
s is an integer or fraction within the range from 0 to 6 and specifies the degree of substitution of XO₄ by Y in the sublattice of the tetrahedral anions,
and
t is an integer or fraction within the range from 0 to 2 and specifies the degree of substitution of Z by Q,
in a quantity that is effective for the absorption of ions from the biological application environment of the dental material.

2. Dental material according to Claim 1,
**characterized in**
**that** the refractive index of filler component A) lies within the range from 1.50 to 1.66.

3. Dental material according to Claim 1 or 2,
**characterized in**
**that** the particle size of filler component A) lies within the range from 0.01 to 10 µm.

4. Dental material according to one or more of the preceding claims,
**characterized in**
**that** A, Band/or B' are the same or different and are Mg²⁺, Ca²⁺, Sr²⁺, Ba²⁺, Cu²⁺, Ag²⁺, Zn²⁺, Na⁺ and/or K⁺.

5. Dental material according to one or more of the preceding claims,
**characterized in**
**that** X is the same or different and is P⁵⁺, Si⁴⁺, B³⁺, Al³⁺ and/or S⁶⁺.

6. Dental material according to one or more of the preceding claims,
**characterized in**
**that** C signifies Na⁺.

7. Dental material according to one or more of the preceding claims,
**characterized in**
**that** the proportion by weight of filler A) mounts to between 1 and 70 wt.%, relative to the dental material.

8. Dental material according to one or more of the preceding claims,
**characterized in**
**that** the proportion by weight of filler A) mounts to between 2 and 35 wt.%, relative to the dental material.

9. Dental material according to one or more of the preceding claims,
**characterized in**
**that** the proportion by weight of filler A) mounts to between 2 and 7 wt.%, relative to the dental material.

10. Dental material according to one or more of the preceding claims,
**characterized in**
**that** it comprises in a mixture with component A)
B1) aspherical, fragmented powders consisting of quartz, glass-ceramics and/or glass having a refractive index from 1.46 to 1.58 and an average particle size form 0.5 to 5.0 µm
and/or
B2) microscopic beads
and/or
B3) inorganic fibers
and/or
B4) organic fibers.

11. Dental material according to Claim 10,
**characterized in**
**that** the weight ratio of A) to B) lies within the range from 10:1 to 1:30.

12. Dental material according to one or more of the preceding claims,
**characterized in**
**that** it comprises as filler C) in the finished completely polymerized dental material globular particles based on SiO₂ consisting of
C1) a spherical, polydisperse and/or monodisperse SiO₂ nucleus which is coated at individual points on its surface with metal-oxide particles, whereby the coated particles exhibit a refractive index from 1.45 to 1.62 and an average primary-particle size from 0.005 to 2 µm and the coating particles consisting of metal oxide have an average size of less than 60 nm,
and/or
C2) SiO₂ having a refractive index between about 1.38 and <1.50 and an average primary particle size from about 0.04 µm to 1.5 µm,
and/or
C3) an SiO₂ nucleus which is coated with an oxide of at least one element of the Groups I, II, III and IV of the Periodic Table, whereby the coated particles exhibit a refractive index from 1.45 to 1.62 and an average primary particle size from 0.04 to 1.5 µm and the coating is between about 15 and 40 nm thick,
and/or
C4) particles described under C1), C2) or C3) which in addition are covered with a of a polymerisable organic binding agent based on monofunctional or multifunctional (meth)acrylates and/or reaction products of isocyanates and methacrylates containing OH groups, whereby the primary particle size of the particles that are provided with the layer of polymerisable binding agent is between about 0.04 and 1.5 µm, whereas thickness of the covering layer is in the range from 5 nm to 50 nm and refractive index of the coated particles lies within the range of 1.40 - 1.52.

13. Dental material according to Claim 12,
**characterized in**
**that** the weight ratio of A) to C) lies within a range from 10:1 to 1:30.

14. Dental material according to one or more of the preceding claims 10 to 13,
**characterized in**
**that** the ratio of filler (B) to filler (C) is adjusted in such a way that an aggregation of the fillers (C) is avoided.

15. Dental material according to one or more of the preceding claims 10 to 14,
**characterized in**
**that** the ratio of (C) to (B) is adjusted within a range from 1:85 to 4:1 in such a way that the strength of the dental material, in terms of its compressive strength, amounts to between > 320 and 480 MPa.

16. Dental material according to one or more of the preceding claims 10 to 15,
**characterized in**
**that** the ratio of (C) to (B) amounts to ≥ 1:10.

17. Dental material according to one or more of the preceding claims 10 to 16,
**characterized in**
**that** the particles C) in the finished completely polymerized material are present separately as discrete particles.

18. Use of one or more mixed apatites of the type
A₁₀₋ᵣ(XO₄)₆₋ₛZ₂₋ₜ - Bᵣ(Y)ₛQₜ - B' ₁₀₋ᵤC_{u'}(Y')₆Z'₂
in which A, B and B' are the same or different and stand for a bivalent cation and/or a charge-equivalent combination of monovalent or trivalent cations, whereby the cations have ionic radii within the range of 0.069 - 0.174 nm,
X being the same or different stands for cations of valency three, four, five or six, whereby the cations have ionic radii within the range of 0.026 - 0.056 nm,
Y and Y' independently of one another are the same or different and signify (SO₄)²⁻ and/or (PO₃F)²⁻ optionally together with (CO₃)²⁻, whereby the proportion of (CO₃)²⁻ is restricted to at most 1/6, relative to the mol of Y and Y',
Q, Z and Z' independently of one another are the same or different and stand for F⁻, OH⁻ and O²⁻ and
C stands for a monovalent cation
and also
u and u' independently of one another are the same or different and are an integer or fraction within the range from 0 to 6 and specify the degree of substitution of B by C in the respective cation sublattice,
r is an integer or fraction within the range from 0 to 10 and specifies the degree of substitution of A by B in the cation sublattice,
s is an integer or fraction within the range from 0 to 6 and specifies the degree of substitution of XO₄ by Y in the sublattice of the tetrahedral anions,
and
t is an integer or fraction within the range from 0 to 2 and specifies the degree of substitution of Z by Q,
in a dental material based on polymerisable, ethylenically unsaturated monomers as binding agent of a catalyst for the cold, hot and/or photopolymerization and relative to the dental material 1 - 95 wt.% of an inorganic filler in a quantity that is effective for the absorption of ions from the biological application environment.

## Revendications

1. Matériau dentaire à base de monomères éthyléniquement insaturés, polymérisables, jouant le rôle de liant, d'un catalyseur pour la polymérisation à froid, à chaud et/ou la photopolymérisation et, par rapport au matériau dentaire, de 1 - 95 % en poids d'une charge inorganique,
**caractérisé en ce que**
la charge A) comporte une ou plusieurs apatites mixtes du type
A₁₀₋ᵣ(XO₄)₆₋ₛZ₂₋ₜ - Bᵣ(Y)ₛQₜ - B'₁₀₋ᵤC_{u'}(Y')₆Z'₂
formule dans laquelle A, B et B' sont identiques ou différents et représentent un cation bivalent et/ou une combinaison de charge équivalente de cations mono- et trivalents, ces cations présentant des rayons ioniques se situant dans la plage de 0,069 - 0,174 nm,
X est identique ou différent et représente des cations tri, tétra, penta et/ou hexavalents, ces cations présentant des rayons ioniques se situant dans la plage de 0,026 - 0,056 nm,
Y et Y' sont, indépendamment l'un de l'autre identiques ou différents et représentent (SO₄)²⁻ et/ou (PO₃F)²⁻, le cas échéant avec (CO₃)²⁻, la partie (CO₃)²⁻ étant limitée au maximum à 1/6^{ème} par rapport aux moles de Y et Y',
Q, Z et Z' sont, indépendamment les uns des autres identiques ou différents et représentent F⁻, OH⁻ et O²⁻, et
C représente un cation monovalent
et
u et u' sont identiques ou différents et représentent, indépendamment l'un de l'autre, un nombre entier ou fractionnaire se situant dans la plage de 0 - 6, qui indique le degré de substitution de B par C dans chaque sous-grille de cations,
r est un nombre entier ou fractionnaire se situant dans la plage de 0 - 10, qui indique le degré de substitution de A par B dans la sous-grille des cations,
s est un nombre entier ou fractionnaire se situant dans la plage de 0 - 6, qui indique le degré de substitution de XO₄ par Y dans la sous-grille des anions tétraédriques,
et
t est un nombre entier ou fractionnaire se situant dans la plage de 0 - 2, qui indique le degré de substitution de Z par Q,
en une quantité efficace pour l'absorption d'ions provenant de l'environnement d'utilisation biologique du matériau dentaire.

2. Matériau dentaire selon la revendication 1,
**caractérisé en ce que**
l'indice de réfraction du composant charge A) se trouve dans la plage de 1,50 à 1,66.

3. Matériau dentaire selon la revendication 1 ou 2,
**caractérisé en ce que**
la grosseur des particules du composant charge A) se trouve dans la plage de 0,01 à 10 µm.

4. Matériau dentaire selon une ou plusieurs des revendications précédentes,
**caractérisé en ce que**
A, B, et/ou B' sont identiques ou différents et représentent Mg²⁺, Ca²⁺, Sr²⁺, Ba²⁺, Cu²⁺, Ag²⁺, Zn²⁺, Na⁺, et/ou K⁺.

5. Matériau dentaire selon une ou plusieurs des revendications précédentes
**caractérisé en ce que**
X représente P⁵⁺, Si⁴⁺, B³⁺, Al³⁺ et/ou S⁶⁺.

6. Matériau dentaire selon une ou plusieurs des revendications précédentes,
**caractérisé en ce que**
C représente Na⁺.

7. Matériau dentaire selon une ou plusieurs des revendications précédentes,
**caractérisé en ce que**
la fraction en poids de charge A) représente 1 à 70 % en poids par rapport au matériau dentaire.

8. Matériau dentaire selon une ou plusieurs des revendications précédentes,
**caractérisé en ce que**
la fraction en poids de charge A) représente 2 à 35 % en poids par rapport au matériau dentaire.

9. Matériau dentaire selon une ou plusieurs des revendications précédentes,
**caractérisé en ce que**
la fraction en poids de charge A) représente 2 à 7 % en poids par rapport au matériau dentaire.

10. Matériau dentaire selon une ou plusieurs des revendications précédentes,
**caractérisé en ce que**
il comporte en mélange avec le composant A)
B1) des poudres asphériques, en forme d'éclats de quartz, céramique de verre et/ou verre d'un indice de réfraction de 1,46 à 1,58 et d'une granulométrie moyenne de 0,5 à 5,0 µm,
et/ou
B2) des micro-sphères
et/ou
B3) des fibres inorganiques
et/ou
B4) des fibres organiques.

11. Matériau dentaire selon la revendication 10,
**caractérisé en ce que**
le rapport en poids de A) par rapport à B) se trouve dans la plage de 10/1 à 1/30.

12. Matériau dentaire selon une ou plusieurs des revendications précédentes,
**caractérisé en ce qu'**
il comporte comme charge C), dans le matériau dentaire polymérisé fini, des particules à base de SiO₂ sphériques, constituées de
C1) un noyau de SiO₂ sphérique, polydispersé et/ou monodispersé, qui est revêtu en des points individuels de sa surface avec des particules d'oxyde métallique, les particules revêtues présentant un indice de réfraction de 1,45 à 1,62 et une grosseur de particules primaires moyenne de 0,005 à 2 µm et les particules de revêtement en oxyde métallique présentent une grosseur moyenne inférieure à 60 nm,
et/ou
C2) SiO₂ d'un indice de réfraction entre environ 1,38 et < 1,50 et une grosseur de particules primaires moyenne d'environ 0,04 µm à 1,5 µm,
et/ou
C3) un noyau de SiO₂ qui est revêtu avec un oxyde d'au moins un élément des groupes I, II, III et IV du Système Périodique, les particules revêtues présentant un indice de réfraction de 1,45 à 1,62 et une grosseur de particules primaires moyenne de 0,04 à 1,5 µm et le revêtement ayant une épaisseur d'environ 15 et 40 nm,
et/ou
C4) des particules décrites en C1), C2, ou C3) qui sont revêtues, en outre, d'une couche d'un liant organique polymérisable, qui repose sur des (méth)acrylates et/ou des produits de réaction d'isocyanates et de méthacrylates contenant des groupes OH, mono- ou multi-fonctionnels, la grosseur de particules primaires des particules pourvues de la couche de liant polymérisable se trouvant entre environ 0,04 et 1,5 µm, tandis que l'épaisseur de la couche de revêtement se situe dans la plage de 5 nm à 50 nm et l'indice de réfraction des particules revêtues se situe dans la plage de 1,40 à 1,52.

13. Matériau dentaire selon la revendication 12,
**caractérisé en ce que**,
le rapport en poids de A) à C) se situe dans la plage de 10/1 à 1/30.

14. Matériau dentaire selon une ou plusieurs des revendications précédentes 10 à 13,
**caractérisé en ce que**
le rapport de la charge (B) à la charge (C) est ajusté de telle sorte qu'une formation d'agrégats de la charge (C) se trouve évitée.

15. Matériau dentaire selon une quelconque des revendications précédentes 10 à 14,
**caractérisé en ce que**
le rapport de (C) à (B) est ajusté dans la plage de 1/85 à 4/1, de telle sorte que la résistance du matériau dentaire par rapport à sa résistance à la compression soit de > 320 à 480 MPa.

16. Matériau dentaire selon une ou plusieurs des revendications précédentes 10 à 15,
**caractérisé en ce que**
le rapport de (C) à (B) est ≥ à 1 /10.

17. Matériau dentaire selon une ou plusieurs des revendications précédentes 10 à 16,
**caractérisé en ce que**
les particules (C) sont présentes dans le matériau dentaire polymérisé fini, séparément, sous forme de particules discrètes.

18. Utilisation d'une ou plusieurs apatites mixtes du type
A₁₀₋ᵣ(XO₄)₆₋ₛZ₂₋ₜ - Bᵣ(Y)ₛQₜ - B'₁₀₋ᵤC_{u'}(Y')₆Z'₂
formule dans laquelle A, B et B' sont identiques ou différents et représentent un cation bivalent et/ou une combinaison de charge équivalente de cations mono- et trivalents, ces cations présentant des rayons ioniques se situant dans la plage de 0,069 - 0,174 nm,
X est identique ou différent et représente des cations tri, tétra, penta et/ou hexavalents, ces cations présentant des rayons ioniques se situant dans la plage de 0,026 - 0,056 nm,
Y et Y' sont identiques ou différents indépendamment l'un de l'autre et représentent (SO₄)²⁻ et/ou (PO₃F)²⁻, le cas échéant avec (CO₃)²⁻, la fraction (CO₃)²⁻ étant limitée au maximum à 1/6^{ième} par rapport aux moles de Y et Y',
Q, Z et Z' sont identiques ou différents indépendamment les uns des autres et représentent F⁻, OH⁻ et O²⁻, et
C représente un cation monovalent
et
u et u' sont identiques ou différents et représentent, indépendamment l'un de l'autre, un nombre entier ou fractionnaire se situant dans la plage de 0 à 6, qui indique le degré de substitution de B par C dans chaque sous-grille de cations,
r est un nombre entier ou fractionnaire se situant dans la plage de 0 à 10, qui indique le degré de substitution de A par B dans une sous-grille des cations,
s est un nombre entier ou fractionnaire se situant dans la plage de 0 à 6, qui indique le degré de substitution de XO₄ par Y dans la sous-grille des anions tétraédriques,
et
t est un nombre entier ou fractionnaire se situant dans la plage de 0 à 2, qui indique le degré de substitution de Z par Q,
dans un matériau dentaire à base de monomères éthyléniquement insaturés, polymérisables, jouant le rôle de liant, d'un catalyseur pour la polymérisation à froid, à chaud et/ou la photopolymérisation et, par rapport au matériau dentaire, de 1 - 95 % en poids d'une charge inorganique en une quantité efficace pour l'absorption d'ions provenant de l'environnement d'utilisation biologique.
